(19) Europäisches Patentamt
European Patent Office
Office européen des brevets

(11) Veröffentlichungsnummer: **0 029 992**
**B1**

(12) # EUROPÄISCHE PATENTSCHRIFT

(45) Veröffentlichungstag der Patentschrift: **29.06.83**

(21) Anmeldenummer: **80107337.0**

(22) Anmeldetag: **25.11.80**

(51) Int. Cl.³: **C 07 D 207/34**, C 07 D 209/42, C 07 D 209/18, C 07 D 213/81, C 07 D 213/82, C 07 D 207/26, A 61 K 31/40, A 61 K 31/405, A 61 K 31/44

(54) **Neue Aminopropanolderivate, Verfahren zu ihrer Herstellung und diese Verbindungen enthaltende Arzneimittel.**

(30) Priorität: **29.11.79 DE 2948056**

(43) Veröffentlichungstag der Anmeldung: **10.06.81 Patentblatt 81/23**

(45) Bekanntmachung des Hinweises auf die Patenterteilung: **29.06.83 Patentblatt 83/26**

(84) Benannte Vertragsstaaten: **AT BE CH DE FR GB IT LI LU NL SE**

(56) Entgegenhaltungen:
**BE-A-706 623**
**CH-A-584 696**
**DE-A-1 493 887**
**DE-A-1 948 795**
**FR-A-2 140 772**
**FR-A-2 361 352**
**FR-A-2 413 382**
**GB-A-1 185 046**
**NL-A-6 818 289**
**US-A-2 274 620**
**US-A-2 652 399**
**US-A-3 661 925**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Friebe, Walter-Gunar, Dr.rer.nat., Heidelberger Landstrasse 111d, D-6100 Darmstadt (DE)**
Erfinder: **Kampe, Wolfgang, Dr.rer.nat., Zedernstrasse 49, D-6805 Heddesheim (DE)**
Erfinder: **Bartsch, Wolfgang, Dr.med.vet., Franconvillerstrasse 5, D-6806 Viernheim (DE)**
Erfinder: **Sponer, Gisbert, Dr.med.vet., Lessingstrasse 13, D-6941 Laudenbach (DE)**
Erfinder: **Dietmann, Karl, Prof.Dr.med., Eisenacher Weg 75, D-6800 Mannheim 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 029 992 B1

ACTORUM AG

# Neue Aminopropanolderivate, Verfahren zu ihrer Herstellung und diese Verbindungen enthaltende Arzneimittel

Die vorliegende Erfindung betrifft neue Aminopropanolderivate der allgemeinen Formel I

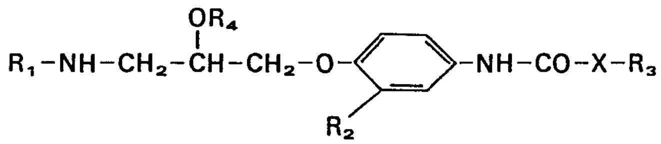
(I)

in welcher

$R_1$ einen $C_1$ bis $C_6$-Alkylrest, der gewünschtenfalls durch eine Gruppe $Z-R_5$ substituiert sein kann,

$R_2$ Wasserstoff oder eine Acetylgruppe,

$R_3$ einen Pyrrolyl-, Pyrrolinyl-, Pyridyl- oder Indolylrest, der gewünschtenfalls ein- oder mehrfach durch $C_1$ bis $C_6$-Alkyl, Hydroxyl, Halogen, Amino oder $C_1$ bis $C_6$-Alkoxy substituiert ist,

$R_4$ Wasserstoff, Acetyl oder Pivaloyl,

$R_5$ einen Indolyl- oder Phenylrest, der gewünschtenfalls ein- oder mehrfach durch Methyl, Methoxy oder Hydroxy substituiert ist,

X einen Valenzstrich oder eine Methylengruppe, und

Z einen Valenzstrich oder ein Sauerstoffatom bedeuten, sowie deren pharmakologisch verträgliche Salze.

Da die Verbindungen der Formel I asymmetrische Kohlenstoffatome besitzen, sind ferner Gegenstand der Erfindung die optisch aktiven Formen und racemische Gemische dieser Verbindungen. Da die Verbindungen der Formel I tautomeriefähige Gruppen enthalten, sind weiterhin Gegenstand der Erfindung sämtliche tautomeren Formen dieser Verbindungen.

Es ist bekannt, dass 1-Amino-3-aryloxy-2-propanolderivate eine β-adrenergische Blockierungsaktivität besitzen. In der niederländischen Patentschrift Nr. 68.18289 sind Derivate mit aliphatischen Resten $R_3$ beschrieben; die deutsche Patentschrift Nr. 1493887 und die britische Patentschrift Nr. 1185046 beanspruchen Substanzen mit aliphatischen und carbocyclischen Resten $R_3$. Ein prominenter Vertreter dieser Verbindungen, Practolol, hat sich mittlerweile als toxisch erwiesen und musste aus dem Verkehr gezogen werden.

Verbindungen mit den hiervorgenannten heterocyclischen Resten $R_3$ sind bisher nicht bekannt geworden. Die Verwendung dieser Reste ruft nun eine überraschende Wirkungssteigerung hervor und bewirkt, dass die Substanzen bisher keine practololartige Toxizität zeigen.

Die Alkylgruppen der Substituenten $R_1$ und $R_3$ sind geradkettige oder verzweigte Gruppen mit 1 bis 6, vorzugsweise 1 bis 4 Kohlenstoffatomen zu verstehen, wie Methyl, Äthyl, Propyl, Isopropyl, Butyl, Isobutyl, sek.-Butyl, tert.-Butyl oder n-Hexyl.

Alkoxygruppen des Substituents $R_3$ enthalten 1 bis 6, vorzugsweise 1 bis 4 Kohlenstoffatome, wie die Methoxy-, Äthoxy-, Propoxy-, Butoxy- oder Pentoxygruppe. Bevorzugt sind die Methoxy-, Äthoxy- und Propoxygruppe.

Unter Halogen wird im Sinne der Erfindung Fluor, Chlor, Brom und Jod verstanden, insbesondere Fluor, Chlor und Brom.

Die Herstellung der neuen Verbindungen der allgemeinen Formel I ist dadurch gekennzeichnet, dass man in an sich bekannter Weise entweder

a) eine Verbindung der Formel II

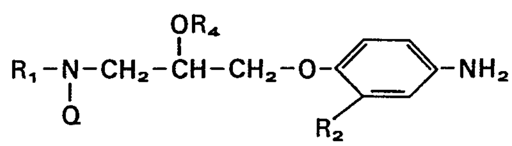
(II)

in welcher $R_1$, $R_2$ und $R_4$ die obengenannte Bedeutung haben, und Q Wasserstoff oder eine Schutzgruppe darstellt, mit einer Verbindung der Formel III

$$Y-CO-X-R_3 \qquad (III)$$

in welcher X und $R_3$ die obengenannte Bedeutung haben, und Y einen reaktiven Rest darstellt, oder

b) eine Verbindung der Formel IV

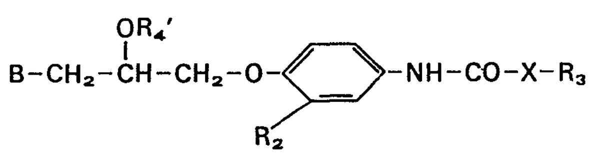
(IV)

in welcher $R_2$, $R_3$ und X die obengenannte Bedeutung haben, B eine reaktive Gruppe darstellt und $R_{4'}$ die oben angegebene Bedeutung für $R_4$ hat oder zusammen mit B einen Valenzstrich bedeutet, mit einer Verbindung der Formel V

$$R_1-NH-Q \qquad (V)$$

in welcher $R_1$ und Q die obengenannte Bedeutung haben, oder

c) eine Verbindung der Formel VI

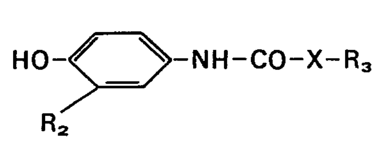
(VI)

in welcher $R_2$, $R_3$ und X die obengenannte Bedeutung haben, mit einer Verbindung der Formel VII

$$R_1-\underset{\underset{Q'}{|}}{N}-CH_2-D-CH_2-B' \qquad (VII)$$

in welcher

$R_1$ die obengenannte Bedeutung hat,

B' eine reaktive Gruppe darstellt,

D für eine CO- oder $CH-OR_{4''}$-Gruppe steht, wobei $R_{4''}$ die obengenannte Bedeutung für $R_4$ hat oder auch zusammen mit B' einen Valenzstrich bedeutet, und

Q' die obengenannte Bedeutung für Q hat oder auch zusammen mit B' eine Einfachbindung sein kann,

umsetzt und, falls D die CO-Gruppe bedeutet, anschliessend reduziert, gegebenenfalls eine für Q oder Q' stehende Schutzgruppe abspaltet, gewünschtenfalls nachträglich in einer erhaltenen Verbindung der allgemeinen Formel I einen Rest $R_4$, nach üblichen Methoden in einen anderen, durch den Anspruch definierten Rest $R_4$ überführt und die erhaltenen Verbindungen gewünschtenfalls in ihre pharmakologisch verträglichen Salze umwandelt.

Für Q oder Q' stehende Schutzgruppen in Verbindungen der Formeln II, V und VII können hydrolytisch oder hydrogenolytisch abspaltbare Reste wie beispielsweise Niederalkanoyl-, Aroyl-, Arylmethyl-, Diarylmethyl- oder Triarylmethylreste sein. Bevorzugt ist der Benzylrest.

Reaktive Reste Y in Verbindungen der allgemeinen Formel III können alle Reste sein, die in der Peptidchemie zur Aktivierung von Carbonsäuren Verwendung finden, beispielsweise Halogenatome, die Azidogruppe, Alkyloxy-, Aryloxy- und Acyloxygruppen.

Reaktive Gruppen B in Verbindungen der allgemeinen Formel IV sowie B' in Verbindungen der allgemeinen Formel VII sind insbesondere Säurereste, beispielsweise von Halogenwasserstoffsäuren und Sulfonsäuren. Besonders bevorzugt sind Chloride, Mesyloxy- und Tosyloxyreste.

Die erfindungsgemässen Verfahren werden zweckmässig in einem unter den Reaktionsbedingungen inerten Lösungsmittel, z.B. Wasser, Methanol, Äthanol, n-Butanol, Dioxan, Dimethylformamid, Hexamethylphosphorsäuretriamid oder Äthylenglykoldimethyläther, gegebenenfalls in Gegenwart eines säurebindenden Mittels durchgeführt. Die Umsetzungen können auch durch Mischen der Reaktionskomponenten ohne Lösungsmittel erreicht werden. Die Reaktionen werden bei Raumtemperatur oder unter Erwärmen, gegebenenfalls unter einer Schutzgasatmosphäre, durchgeführt.

Die gegebenenfalls durchzuführende Reduktion der für D stehenden Gruppe CO erfolgt zweckmässig durch katalytische Hydrierung mit Edelmetall- oder Nickelkatalysatoren oder mittels komplexer Metallhydride wie z.B. Natriumborhydrid.

Die bei den erfindungsgemässen Verfahren eingesetzten Ausgangsverbindungen sind in der Regel literaturbekannte Verbindungen. Neue Verbindungen werden im allgemeinen analog der für die Herstellung dieser bekannten Verbindungen beschriebenen Verfahren erhalten.

Als gegebenenfalls nachträglich durchzuführende Umwandlung eines Substituenten $R_4$ kommt eine nachträgliche Acetylierung bzw. Pivaloylierung der OH-Gruppe in Frage. Die Veresterung kann in an sich üblicher Weise durch Umsetzung mit einem Säurehalogenid oder Säureanhydrid, gegebenenfalls in Gegenwart eines säurebindenden Mittels wie beispielsweise Pyridin oder Triäthylamin erfolgen.

Die Abspaltung einer für Q oder Q' stehenden Arylmethyl-, Diarylmethyl- oder Triarylmethylgruppe erfolgt beispielsweise durch Hydrierung in Gegenwart von Edelmetallkatalysatoren.

Zur Überführung der Verbindungen der Formel I in ihre pharmakologisch unbedenklichen Salze setzt man diese vorzugsweise in einem organischen Lösungsmittel mit einer anorganischen oder organischen Säure, z.B. Salzsäure, Bromwasserstoffsäure, Phosphor-, Schwefel-, Essig-, Milch-, Zitronen-, Malein oder Benzoesäure um.

Die erfindungsgemässen Verbindungen der Formel I können in Form eines racemischen Gemisches anfallen. Die Trennung des Racemats in die optisch aktiven Formen geschieht nach an sich bekannten Methoden über die diastereomeren Salze. Als aktive Säuren können vorwiegend Wein-, Apfel-, Campher- und Camphersulfonsäure verwendet werden.

Zur Herstellung von Arzneimitteln werden die Verbindungen der Formel I in an sich bekannter Weise mit geeigneten pharmazeutischen Trägersubstanzen, Aroma-, Geschmacks- und Farbstoffen gemischt und beispielsweise als Tabletten oder Dragées ausgeformt oder unter Zugabe entsprechender Hilfsstoffe in Wasser oder Öl, wie z.B. Olivenöl, suspendiert oder gelöst.

Die erfindungsgemässen neuen Verbindungen der Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Äthanol, Komplexbildner (wie Äthylendiamintetraessigsäure und deren nichttoxische Salze), hochmolekulare Polymere (wie flüssiges Polyäthylenoxyd) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie Polyäthylenglykole); für orale Applikation geeignete Zubereitungen können gewünschtenfalls Geschmacks- und Süssstoffe enthalten.

Die verabreichte Dosierung hängt vom Alter, der Gesundheit und dem Gewicht des Empfängers, dem Ausmass der Krankheit, der Art gleichzeitiger gegebenenfalls durchgeführter weiterer Behandlungen, der Häufigkeit der Behandlungen und der Art der gewünschten Wirkung ab. Üblicherweise

beträgt die tägliche Dosis der aktiven Verbindung 0,1 bis 50 mg/kg Körpergewicht. Normalerweise sind 0,5 bis 40, und vorzugsweise 1,0 bis 20 mg/kg/d in einer oder mehreren Anwendungen pro Tag wirksam, um die gewünschten Resultate zu erhalten.

Zum Nachweis der β-adrenergischen Blockierungsaktivität wurden folgende Versuche durchgeführt:

*Versuchsprotokoll*

Kaninchen wurden in Holzkäfigen fixiert und die Herzfrequenz über Stichelektroden abgeleitet und auf einem Frequenzzählgerät (Messzeit 15 s) abgelesen. Über eine Ohrvene wurde zunächst 1 mg/kg i.v. Isoprenalin injiziert, was eine Erhöhung der Herzfrequenz von ca. 210 auf ca. 350 Schläge/min bewirkte. Anschliessend wurden die gelösten Prüfsubstanzen in steigender Dosierung im Abstand von 10 min (0,125 + 0,125 + 0,250 + 0,500 + 1,0 + 2,0 + 4,0 mg/kg i.v.) intravenös verabreicht und die Herzfrequenz nach Isoprenalingabe erneut abgelesen. Die Hemmung der Isoprenalintachycardie ist als Mass für die β-Blockade anzusehen. Es wurde die Dosis der Prüfsubstanzen interpoliert, die den Anstieg der Herzfrequenz unter Isoprenalin (1 µg/kg i.v.) um 30% abschwächt ($HD_{30}$).

Als Referenzsubstanz wurde der chemisch ähnlich strukturierte bekannte β-Blocker Practolol (ehemals als Dalzic® im Handel) verwendet.

Die Ergebnisse sind in Tabelle I zusammengestellt.

*Tabelle I*

| Substanz | $HD_{30}$* (µg/kg i.v.) |
|---|---|
| Practolol | 1320 |
| Beispiel 1 | 469 |
| Beispiel 3 | 777 |
| Beispiel 2a | 1124 |
| Beispiel 2b | 3413 |
| Beispiel 2c | 2093 |
| Beispiel 2d | 824 |
| Beispiel 4 | 1761 |

*Tabelle I (Fortsetzung)*

| Substanz | $HD_{30}$* (µg/kg i.v.) |
|---|---|
| Beispiel 2e | 308 |
| Beispiel 7 | 1454 |
| Beispiel 2f | 7697 |
| Beispiel 8a | 3549 |
| Beispiel 8b | 1676 |
| Beispiel 2g | >8000 |

* $HD_{30}$ = Mass für die β-blockierende Wirksamkeit. Dosis zur Hemmung des Frequenzanstieges unter 1 µg/kg i.v. Isoprenalin um 30%.

Die nachfolgenden Beispiele zeigen einige der zahlreichen Verfahrensvarianten, die zur Synthese der erfindungsgemässen Verbindungen verwendet werden können, sowie ein Tablettierungsbeispiel.

*Beispiel 1:*

*1-[4-(Indol-2-carboxamido)phenoxy]-3-isopropylamino-2-propanolhydrochlorid*

Zu der Mischung aus 18,8 g 1-(4-Aminophenoxy)-3-N-benzylisopropylamino-2-propanol, 33,5 g Natriumhydrogencarbonat und 200 ml Dichlormethan tropft man die Lösung von 13,4 g Indol-2-carbonylchlorid in 50 ml Dichlormethan, erwärmt 5 h zum Rückfluss, versetzt mit verd. Natronlauge, trennt die organische Phase ab, engt ein und verreibt ein Rückstand mit Äther. Es verbleiben 17,6 g 1-[4-(Indol-2-carboxamido)-phenoxy]-3-N-benzylisopropylamino-2-propanol vom Schmp. 173-175° C.

Die vorstehende Verbindung wird in Methanol gelöst, mit ätherischer Chlorwasserstofflösung versetzt, eingeengt, der Rückstand in 200 ml 90%ig. Methanol aufgenommen und bei Raumtemperatur und 1 bar Wasserstoffdruck über 2 g 10%ig. Palladiumkohle hydriert. Nach Aufnahme der berechneten Wasserstoffmenge wird filtriert, die Lösung eingeengt und der Rückstand aus Äthanol umkristallisiert. Man erhält 12,5 g Titelverbindung (48% d.Th.) vom Schmp. 247-249° C.

*Beispiel 2*

In analoger Weise wie in Beispiel 1 beschrieben erhält man:

| | Bezeichnung | Ausbeute (%) | Schmelzpunkt (°C) (Lösungsmittel) |
|---|---|---|---|
| a) | 1-[2-(3,4-Dimethoxyphenyl)äthylamino]-3-[4-(indol-2-carboxamido)phenoxy]-2-propanolhydrochlorid aus 1-(4-Aminophenoxy)-3-[N-benzyl-2-(3,4-dimethoxyphenyl)-äthylamino]-2-propanol und Indol-2-carbonylchlorid | 62 | 222-223 (Methanol) |
| b) | 1-[4-(Indol-2-carboxamido)phenoxy]-3-[2-(2-methoxy-phenoxy)äthylamino]-2-propanolhydrochlorid aus 1-(4-Aminophenoxy)-3-[N-benzyl-2-(2-methoxyphenoxy)äthyl-amino]-2-propanol und Indol-2-carbonylchlorid | 57 | 160-162 (Methanol) |

*Beispiel 2 (Fortsetzung)*

| | Bezeichnung | Ausbeute (%) | Schmelzpunkt (°C) (Lösungsmittel) |
|---|---|---|---|
| c) | 1-[4-(Indol-2-carboxamido)phenoxy]-3-[(+)-(S)-1-methylpropylamino]-2-propanolhydrochlorid aus 1-(4-Aminophenoxy)-3-[(+)-(S)-N-benzyl-1-methylpropylamino]-2-propanol und Indol-2-carbonylchorid | 50 | 216-217 (Äthanol/Äther) |
| d) | 1-Isopropylamino-3-(4-nicotinoylamidophenoxy)-2-propanolhydrochlorid aus 1-(4-Aminophenoxy)-3-N-benzylisopropylamino-2-propanol und Nicotinoylchlorid | 86 | 227-228 (Methanol/Äther) |
| e) | 1-Isopropylamino-3-[4-(pyrrol-2-carboxamido)phenoxy]-2-propanol aus 1-(4-Aminophenoxy)-3-N-benzylisopropylamino-2-propanol und Pyrrol-2-carbonylchlorid | 59 | 177-178 (Essigester) |
| f) | 1-[4-(5-n-Butylpyridin-2-carboxamido)phenoxy]-3-isopropylamino-2-propanolhydrochlorid aus 1-(4-Aminophenoxy)-3-N-benzylisopropylamino-2-propanol und 5-n-Butylpyridin-2-carbonylchlorid | 89 | 160-162 (Isopropanol) |
| g) | 1-[4-(2-Hydroxypyridin-3-carboxamido)phenoxy]-3-isopropylamino-2-propanolhydrochlorid aus 1-(4-Aminophenoxy)-3-N-benzylisopropylamino-2-propanol und 2-Hydroxynicotinoylchlorid | 50 | 275-276 (Methanol) |
| h) | 1-t-Butylamino-3-[4-(indol-2-carboxamido)phenoxy]-2-propanolhydrochlorid aus 1-(4-Aminophenoxy)-3-N-benzyl-t-butylamino-2-propanol und Indol-2-carbonylchlorid | 54 | 268-270 (Methanol) |

*Beispiel 3:*

*1-[4-(Indol-3-acetamido)phenoxy]-3-isopropylamino-2-propanol*

Eine Mischung aus 8,75 g Indol-3-essigsäure, 7 ml Triäthylamin, 150 ml Dioxan und 75 ml Aceton wird 10 min zum Rückfluss erwärmt, auf −5° C abgekühlt und mit der Lösung von 13 ml Chlorameisensäureisobutylester in 60 ml Dioxan und 30 ml Aceton versetzt. Nach 10 min Rühren tropft man eine Lösung von 15,7 g 1-(4-Aminophenoxy)-3-N-benzylisopropylamino-2-propanol und 7 ml Triäthylamin in 40 ml Wasser, 40 ml Dioxan und 20 ml Aceton zu, rührt 15 min bei 0° C, giesst in Wasser und extrahiert mit Dichlormethan. Der Extrakt wird eingeengt und durch Säulenchromatographie an Kieselgel, Elutionsmittel Dichlormethan/Methanol 97:3, gereinigt.

Man isoliert 9,8 g 1-[4-(Indol-3-acetamido)-phenoxy]-3-N-benzylisopropylamino-2-propanol, die wie in Beispiel 1 beschrieben hydrogenolytisch debenzyliert werden.

Nach Überführung in die freie Base und Umkristallisation aus Essigester/Äther verbleiben 6,0 g Titelverbindung (32% d.Th.) vom Schmp. 137-138° C.

*Beispiel 4:*

*1-Isopropylamino-3-[4-(4,5-dihydro-2-hydroxypyrrol-1-acetamido)phenoxy]-2-propanol*

Eine Mischung aus 9,3 g 1-(4-Aminophenoxy)-3-N-benzylisopropylamino-2-propanol und 6,0 g 4,5-Dihydro-2-hydroxypyrrol-1-essigsäureäthylester wird 16 h auf 100° C erhitzt und anschliessend durch Säulenchromatographie an Kieselgel, Elutionsmittel Dichlormethan/Methanol 19:1, gereinigt.

Man isoliert 9,5 g 1-N-Benzylisopropylamino-3-[4-(4,5-dihydro-2-hydroxypyrrol-1-acetamido)phenoxy]-2-propanol, die in einem Lösungsmittelgemisch aus 100 ml Methanol und 100 ml Tetrahydrofuran bei 1 bar Wasserstoffdruck über 1 g 10%ig. Palladiumkohle hydrogenolytisch debenzyliert werden.

Nach Filtration, Einengen und Umkristallisation aus Isopropanol/Äther verbleiben 5,2 g Titelverbindung (49% d.Th.) vom Schmp. 123-124° C.

*Beispiel 5:*

*1-[4-(Indol-2-carboxamido)phenoxy]-3-isopropylamino-2-propanol*

Zu der Lösung von 11,1 g 1-(4-Aminophenoxy)-3-isopropylamino-2-propanol in 200 ml 50%ig. wässerigem Aceton tropft man bei pH 4 bis 5 gleichzeitig die Lösung von 13,5 g Indol-2-carbonylchlorid in 50 ml Aceton und verdünnte Natronlauge, so dass der pH-Wert der Lösung zwischen 4 und 5 gehalten wird. Nach 1 h Rühren bei Raumtemperatur wird mit Natronlauge auf pH 11 bis 12 eingestellt und filtriert. Man isoliert nach Auswaschen mit Aceton und Äther 16,3 g Titelverbindung (89% d.Th) vom Schmp. 208-210° C.

*Beispiel 6*

In analoger Weise wie in Beispiel 5 beschrieben erhält man:

| | Bezeichnung | Ausbeute (%) | Schmelzpunkt (°C) (Lösungsmittel) |
|---|---|---|---|
| a) | 1-[4-(Indol-3-carboxamido)phenoxy]-3-isopropylamino-2-propanol aus 1-(4-Aminophenoxy)-3-isopropylamino-2-propanol und Indol-3-carbonylchlorid | | |
| b) | 1-[4-(5-Fluorindol-2-carboxamido)phenoxy]-3-isopropylamino-2-propanol aus 1-(4-Aminophenoxy)-3-isopropylamino-2-propanol und 5-Fluorindol-2-carbonylchlorid | 68 | 195-197 (Isopropanol) |
| c) | 1-[4-(4-Hydroxyindol-2-carboxamido)phenoxy]-3-isopropylamino-2-propanol aus 1-(4-Aminophenoxy)-3-isopropylamino-2-propanol und 4-Benzylindol-2-carbonylchlorid | 62 | 205-207 (Äthanol) |
| d) | 1-Isopropylamino-3-[4-(5-methoxyindol-2-carboxamido)-phenoxy]-2-propanol aus 1-(4-Aminophenoxy)-3-isopropylamino-2-propanol und 5-Methoxyindol-2-carbonylchlorid | 75 | 165-167 (Isopropanol) |
| e) | 1-[4-(4,5-Dimethylindol-2-carboxamido)-phenoxy]-3-isopropylamino-2-propanol aus 1-(4-Aminophenoxy)-3-isopropylamino-2-propanol und 4,5-Dimethylindol-2-carbonylchlorid | 95 | 175-177 (Äthanol) |
| f) | 1-Isopropylamino-3-[4-(5-methoxy-4-methylindol-2-carboxamido)phenoxy]-2-propanol aus 1-(4-Aminophenoxy)-3-isopropylamino-2-propanol und 5-Methoxy-4-methylindol-2-carbonylchlorid | 76 | 205-207 (Äthanol) |
| g) | 1-[4-(6-Hydroxypyridin-2-carboxamido)phenoxy]-3-isopropylamino-2-propanolhydrochlorid aus 1-(4-Aminophenoxy)-3-N-benzylisopropylamino-2-propanol und 6-Hydroxypyridin-2-carbonylchlorid | 69 | 189-192 (Äthanol) |
| h) | 1-[4-(5-Brom-2-methoxynicotinoylamido)phenoxy]-3-isopropylamino-2-propanolhydrochlorid aus 1-(4-Aminophenoxy)-3-isopropylamino-2-propanol und 5-Brom-2-methoxynicotinsäurechlorid | 50 | 212-214 (Aceton) |
| i) | 1-[4-(7-Chlorindol-2-carboxamido)phenoxy]-3-isopropylamino-2-propanol aus 1-(4-Aminophenoxy)-3-isopropylamino-2-propanol und 7-Chlorindol-2-carbonylchlorid | 72 | 165-168 (Essigester) |

*Beispiel 7:*

*1-[2-Acetyl-4-(indol-2-carboxamido)phenoxy]-3-isopropylamino-2-propanol*

Eine Mischung aus 10,0 g 2-(2,3-Epoxypropoxy)-5-(Indol-2-carboxamido)acetophenon, 100 ml Äthanol und 75 ml Isopropylamin wird 24 h zum Rückfluss erhitzt, eingeengt und durch Säulenchromatographie an Kieselgel, Elutionsmittel Dichlormethan/Methanol 4:1, gereinigt.

Man isoliert 3,7 g Titelverbindung (30% d.Th.) vom Schmp. 190-210° C (als amorphes Hydrochlorid).

Das als Ausgangsstoff verwendete 2-(2,3-Epoxypropoxy)-5-(indol-2-carboxamido)acetophenon kann wie folgt erhalten werden:

Die Reaktion von 5-Amino-2-hydroxyacetophenon mit Indol-2-carbonsäurechlorid führt zu 2-Hydroxy-5-(indol-2-carboxamido)acetophenon vom Schmp. 119° C, das mit Epichlorhydrin in Gegenwart von Natriumalkoholat in das rohe 2-(2,3-Epoxypropoxy)-5-(indol-2-carboxamido)acetophenon umgesetzt wird.

*(Beispiel 8 auf der nächsten Seite)*

*Beispiel 9:*

*1-[4-(Indol-2-carboxamido)phenoxy]-3-isopropylamino-2-pivaloyloxy-propan*

4,6 g 1-[4-(Indol-2-carboxamido)phenoxy]-3-isopropylamino-2-propanol (freie Base der Verbindung nach Beispiel 1) werden zu 30 g geschmolzener Pivalinsäure gegeben und mit 6,3 g Pivalinsäureanhydrid versetzt. Man rührt 5 Tage bei Raumtemperatur, giesst in 100 ml Eiswasser, neutralisiert mit verd. Ammoniak (1:10), extrahiert mit Dichlormethan und engt den Extrakt ein. Man erhält die Titelverbindung (88% d.Th.) vom Schmp. 138-140° C.

*Beispiel 8*

In analoger Weise wie in Beispiel 7 beschrieben erhält man:

| | Bezeichnung | Ausbeute (%) | Schmelzpunkt (°C) (Lösungsmittel) |
|---|---|---|---|
| a) | 1-[1,1-Dimethyl-2-(4-hydroxyphenyl)ethylamino]-3-[4-(indol-2-carboxamido)phenoxy]-2-propanol aus N-[4-(2,3-Epoxypropoxy)phenyl]-indol-2-carboxamid und 1,1-Dimethyl-2-(4-hydroxyphenyl)ethylamin | 44 | 150-152 (Aceton) |
| b) | 1-[4-(Indol-2-carboxamido)phenoxy]-3-[1,1-dimethyl-2-(3-indolyl)ethylamino]-2-propanol aus N-[4-(2,3-Epoxypropoxy)phenyl]-indol-2-carboxamid und 1,1-Dimethyl-2-(3-indolyl)ethylamin | 37 | 153-155 (Essigester) |

*Beispiel 10:*

Es wurden Tabletten hergestellt: Jede Tablette enthält 10 mg 1-[4-(Indol-2-carboxamido)-phenoxy]-3-isopropylamino-2-propanol-hydrochlorid. Die Tabletten wurden gemäss der folgenden Rezeptur hergestellt:

| | |
|---|---|
| 1-[4-(Indol-2-carboxamido)phenoxy]-3-isopropylamino-2-propanolhydrochlorid | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpresst.

**Patentansprüche**

1. Aminopropanolderivate der allgemeinen Formel (1)

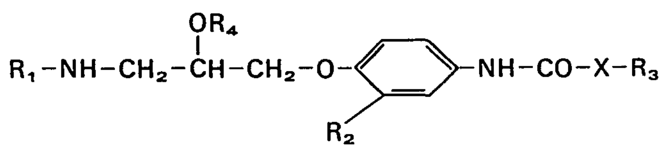

(I)

in welcher

$R_1$ einen $C_1$ bis $C_6$-Alkylrest, der gewünschtenfalls durch eine Gruppe $Z-R_5$ substituiert sein kann,

$R_2$ Wasserstoff oder eine Acetylgruppe,

$R_3$ einen Pyrrolyl-, Pyrrolinyl-, Pyridyl- oder Indolylrest, der gewünschtenfalls ein- oder mehrfach durch $C_1$ bis $C_6$-Alkyl, Hydroxyl, Halogen, Amino oder $C_1$ bis $C_6$-Alkoxy substituiert ist,

$R_4$ Wasserstoff, Acetyl oder Pivaloyl,

$R_5$ einen Indolyl- oder Phenylrest, der gewünschtenfalls ein- oder mehrfach durch Methyl, Methoxy oder Hydroxy substituiert ist,

X einen Valenzstrich oder eine Methylengruppe, und

Z einen Valenzstrich oder ein Sauerstoffatom bedeuten, sowie deren pharmakologisch verträgliche Salze.

2. Verbindungen gemäss Anspruch 1, worin $R_3$ einen gegebenenfalls ein- oder mehrfach durch Halogen, $C_1$ bis $C_6$-Alkyl oder $C_1$ bis $C_6$-Alkoxy substituierten Indolylrest bedeutet, sowie deren pharmakologisch verträgliche Salze.

3. Verbindungen gemäss Anspruch 2, worin $R_3$ einen Indol-2-ylrest bedeutet, sowie deren pharmakologisch verträgliche Salze.

4. Verbindungen gemäss Anspruch 1, worin $R_3$ einen Pyrrolylrest bedeutet, sowie deren pharmakologisch verträgliche Salze.

5. Verbindungen gemäss Anspruch 1, worin $R_3$ einen Pyridylrest bedeutet, sowie deren pharmakologisch verträgliche Salze.

6. Verbindungen gemäss den Ansprüchen 1 bis 5, worin $R_1$ eine Isopropylgruppe bedeutet, sowie deren pharmakologisch verträgliche Salze.

7. Verfahren zur Herstellung von Aminopropanolderivaten der allgemeinen Formel (I)

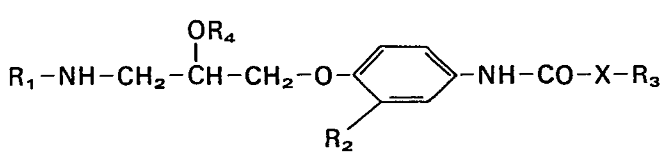

(I)

in welcher

$R_1$ einen $C_1$ bis $C_6$-Alkylrest, der gewünschtenfalls durch eine Gruppe $Z-R_5$ substituiert sein kann,

$R_2$ Wasserstoff oder eine Acetylgruppe,

$R_3$ einen Pyrrolyl-, Pyrrolinyl-, Pyridyl- oder Indolylrest, der gewünschtenfalls ein- oder mehrfach durch $C_1$ bis $C_6$-Alkyl, Hydroxyl, Halogen, Amino oder $C_1$ bis $C_6$-Alkoxy substituiert ist,

$R_4$ Wasserstoff, Acetyl oder Pivaloyl,

$R_5$ einen Indolyl- oder Phenylrest, der gewünschtenfalls ein- oder mehrfach durch Methyl, Methoxy oder Hydroxy substituiert ist,

X einen Valenzstrich oder Methylengruppe, und

Z einen Valenzstrich oder ein Sauerstoffatom bedeuten, sowie deren pharmakologisch verträgliche Salze, dadurch gekennzeichnet, dass man in an sich bekannter Weise entweder

a) eine Verbindung der Formel (II)

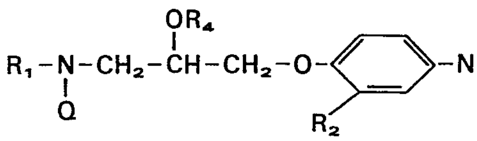 (II)

in welcher $R_1$, $R_2$ und $R_4$ die obengenannte Bedeutung haben und Q Wasserstoff oder eine Schutzgruppe darstellt, mit einer Verbindung der Formel (III)

$$Y-CO-X-R_3 \quad (III)$$

in welcher X und $R_3$ die obengenannte Bedeutung haben und Y einen reaktiven Rest darstellt, oder

b) eine Verbindung der Formel (IV)

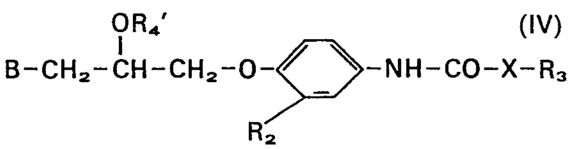 (IV)

in welcher $R_2$, $R_3$ und X die obengenannte Bedeutung haben, B eine reaktive Gruppe darstellt und $R_{4'}$ die oben angegebene Bedeutung für $R_4$ hat, oder zusammen mit B einen Valenzstrich bedeutet, mit einer Verbindung der Formel (V)

$$R_1-NH-Q \quad (V)$$

in welcher $R_1$ und Q die obengenannte Bedeutung haben, oder

c) eine Verbindung der Formel (VI)

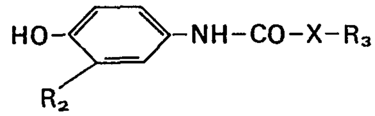 (VI)

in welcher $R_2$, $R_3$ und X die obengenannte Bedeutung haben, mit einer Verbindung der Formel (VII)

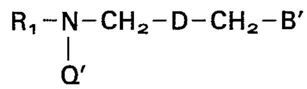 (VII)

in welcher

$R_1$ die obengenannte Bedeutung hat,

B' eine reaktive Gruppe darstellt,

D für eine CO– oder $CH-OR_{4''}$-Gruppe steht, wobei $R_{4''}$ die obengenannte Bedeutung für $R_4$ hat, oder auch zusammen mit B' einen Valenzstrich bedeutet, und

Q' die obengenannte Bedeutung für Q hat oder auch zusammen mit B' eine Einfachbindung sein kann, umsetzt und, falls D die CO-Gruppe bedeutet, anschliessend reduziert, gegebenenfalls eine für Q oder Q' stehende Schutzgruppe abspaltet, gewünschtenfalls nachträglich in einer erhaltenen Verbindung der allgemeinen Formel (I) einen Rest $R_4$ nach üblichen Methoden in einen anderen, durch den Anspruch 1 definierten Rest $R_4$ überführt und die erhaltenen Verbindungen gewünschtenfalls in ihre pharmakologisch verträglichen Salze umwandelt.

8. Verbindungen gemäss den Ansprüchen 1 bis 6 zur Bekämpfung von Herzerkrankungen.

9. Arzneimittel, gekennzeichnet durch einen Gehalt an Verbindungen der allgemeinen Formel (I) gemäss Ansprüche 1 bis 6 bzw. deren pharmakologisch verträgliche Salze.

## Claims

1. Aminopropanol derivatives of the general formula (I)

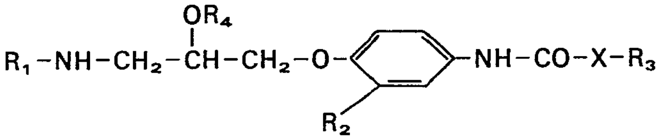 (I)

in which $R_1$ signifies a $C_1$-$C_6$ alkyl radical which, if desired, can be substituted by a group $Z-R_5$, $R_2$ hydrogen or an acetyl group, $R_3$ a pyrrolyl, pyrrolinyl, pyridyl or indolyl radical which, if desired, is substituted one or more times by $C_1$-$C_6$ alkyl, hydroxyl, halogen, amino or $C_1$-$C_6$ alkoxy, $R_4$ hydrogen, acetyl or pivaloyl, $R_5$ an indolyl or phenyl radical which, if desired, is substituted one or more times by methyl, methoxy or hydroxyl, X a valency bond or a methylene group, and Z a valency bond or an oxygen atom, as well as their pharmacologically acceptable salts.

2. Compounds according to claim 1, wherein $R_3$ signifies an indolyl radical possibly substituted one or more times by halogen, $C_1$-$C_6$ alkyl or $C_1$-$C_6$ alkoxy, as well as their pharmacologically acceptable salts.

3. Compounds according to claim 2, wherein $R_3$ signifies an indol-2-yl radical, as well as their pharmacologically acceptable salts.

4. Compounds according to claim 1, wherein $R_3$ signifies a pyrrolyl radical, as well as their pharmacologically acceptables salts.

5. Compounds according to claim 1, wherein $R_3$ signifies a pyridyl radical, as well as their pharmacologically acceptable salts.

6. Compounds according to claims 1 to 5, wherein $R_1$ signifies an isopropyl group, as well as their pharmacologically acceptable salts.

7. Process for the preparation of aminopropanol derivatives of the general formula (I)

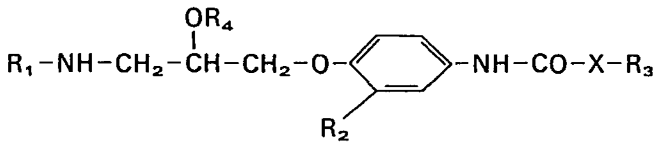
(I)

in which $R_1$ signifies a $C_1$-$C_6$ alkyl radical which, if desired, can be substituted by a group $Z-R_5$, $R_2$ hydrogen or an acetyl group, $R_3$ a pyrrolyl, pyrrolinyl, pyridyl or indolyl radical which, if desired, is substituted one or more times by $C_1$-$C_6$ alkyl, hydroxyl, halogen, amino or $C_1$-$C_6$ alkoxy, $R_4$ hydrogen, acetyl or pivaloyl, $R_5$ an indolyl or phenyl radical which, if desired, is substituted one or more times by methyl, methoxy or hydroxyl, X a valency bond or methylene group, and Z a valency bond or an oxygen atom, as well as their pharmacologically acceptable salts, characterised in that, in *per se* known manner, one either

a) reacts a compound of the formula (II)

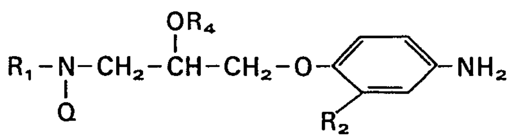
(II)

in which $R_1$, $R_2$ and $R_4$ have the above-mentioned meaning and Q represents hydrogen or a protective group, with a compound of the formula (III)

$$Y-CO-X-R_3 \qquad (III)$$

in which X and $R_3$ have the above-mentionned meaning and Y represents a reactive residue, or

b) reacts a compound of the formula (IV)

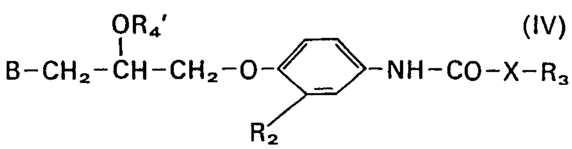

in which $R_2$, $R_3$ and X have the above-mentioned meaning, B represents a reactive group, and $R_{4'}$ has the above-given meaning for $R_4$ or, together with B, signifies a valency bond, with a compound of the formula (V)

$$R_1-NH-Q \qquad (V)$$

in which $R_1$ and Q have the above-mentioned meaning, or

c) reacts a compound of the formula (VI)

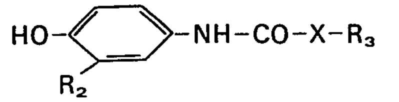
(VI)

in which $R_2$, $R_3$ and X have the above-mentioned meaning, with a compound of the formula (VII)

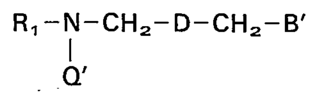
(VII)

in which $R_1$ has the above-mentioned meaning, B' represents a reactive group, D stands for a $-CO-$ or $-CH-OR_{4''}$ group, whereby $R_{4''}$ has the above-mentioned meaning for $R_4$ or also, together with B', signifies a valency bond, and Q' has the above-mentioned meaning for Q or also, together with B', can be a single bond, and, if D signifie the $-CO-$ group, subsequently reduces, possibly splits off a protective group standing for Q or Q', if desired, in a compound obtained of the general formula (I), converts a radical $R_4$ according to usual methods into another radical $R_4$ defined by claim 1 and, if desired, converts the compounds obtained into their pharmacologically acceptable salts.

8. Compounds according to claims 1 to 6 for the combating of heart diseases.

9. Medicaments, characterised by a content of compounds of the general formula (I) according to claims 1 to 6 or of their pharmacologically acceptable salts.

**Revendications**

1. Dérivés d'aminopropanol de formule générale (I)

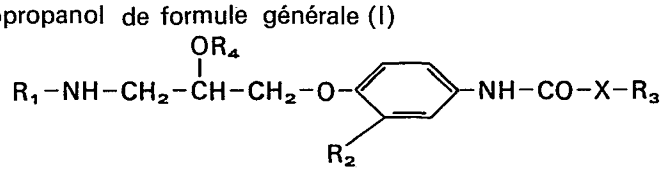
(I)

dans laquelle

$R_1$ est un reste alkyle $C_1$-$C_6$, pouvant être éventuellement substitué par un groupe $Z-R_5$

$R_2$ est de l'hydrogène ou un groupe acétyle,

$R_3$ est un reste pyrrolyle, pyrrolinyle, pyridyle ou indolyle éventuellement substitué une ou plusieurs fois par alkyle $C_1$-$C_6$, hydroxyle, halogène, amino ou alcoxy $C_1$-$C_6$,

$R_4$ est de l'hydrogène, de l'acétyle ou du pivaloyle,

$R_5$ est un reste indolyle ou phényle, éventuellement substitué une ou plusieurs fois par méthyle, méthoxy ou hydroxyle,

X est un trait de valence ou un groupe méthylène, et

Z est un trait de valence ou un atome d'oxygène, ainsi que leurs sels pharmacologiquement tolérables.

2. Composés selon la revendication 1, caractérisés en ce que $R_3$ est un reste indolyle éventuellement substitué une ou plusieurs fois par de l'halogène, de l'alkyle $C_1$-$C_6$ ou de l'alcoxy $C_1$-$C_6$, ainsi que leurs sels pharmacologiquement tolérables.

3. Composés selon la revendication 2, caractérisés en ce que $R_3$ est un reste indole-2-yle, ainsi que leurs sels pharmacologiquement tolérables.

4. Composés selon la revendication 1, caractérisés en ce que $R_3$ est un reste pyrrolyle, ainsi que leurs sels pharmacologiquement tolérables.

5. Composés selon la revendication 1, caractérisés en ce que $R_3$ est un reste pyridyle, ainsi que leurs sels pharmacologiquement tolérables.

6. Composés selon les revendications 1 à 5, caractérisés en ce que $R_1$ est un groupe isopropyle, ainsi que leurs sels pharmacologiquement tolérables.

7. Procédé de préparation de dérivés d'aminopropanol de formule générale (I)

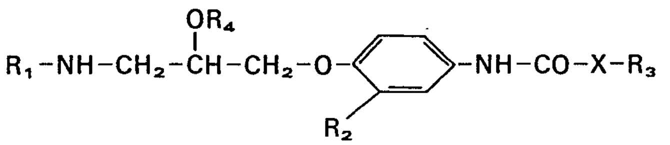

(I)

dans laquelle

$R_1$ est un reste alkyle $C_1$-$C_6$, pouvant être éventuellement substitué par un groupe $Z-R_5$

$R_2$ est de l'hydrogène ou un groupe acétyle,

$R_3$ est un reste pyrrolyle, pyrrolinyle, pyridyle ou indolyle éventuellement substitué une ou plusieurs fois par alkyle $C_1$-$C_6$, hydroxyle, halogène, amino ou alcoxy $C_1$-$C_6$,

$R_4$ est de l'hydrogène, de l'acétyle ou du pivaloyle,

$R_5$ est un reste indolyle ou phényle, éventuellement substitué une ou plusieurs fois par méthyle, méthoxy ou hydroxyle,

X est un trait de valence ou un groupe méthylène, et

Z est un trait de valence ou un atome d'oxygène, ainsi que leurs sels pharmacologiquement tolérables, caractérisé en ce que, de façon en soi connue, on fait réagir

soit:

a) un composé de formule (II)

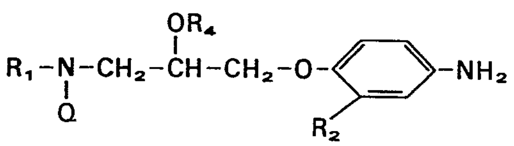

(II)

dans laquelle $R_1$, $R_2$ et $R_4$ ont la signification indiquée ci-dessus et Q est de l'hydrogène ou un groupe de protection, avec un composé de formule (III)

$$Y-CO-X-R_3 \qquad (III)$$

dans laquelle X et $R_3$ ont la signification donnée ci-dessus et Y est un reste réactif,

soit:

b) un composé de formule (IV)

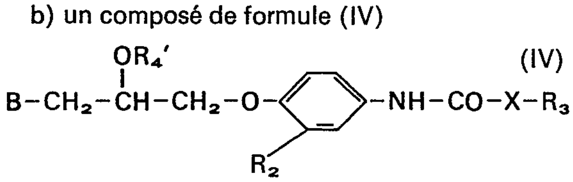

(IV)

dans laquelle $R_2$, $R_3$ et X ont la signification ci-dessus, B est un groupe réactif et $R'_4$ a la signification donnée ci-dessus pour $R_4$ ou représente ensemble avec B un trait de valence, avec un composé de formule (V)

$$R_1-NH-Q \qquad (V)$$

dans laquelle $R_1$ et Q ont la signification donnée ci-dessus,

soit:

c) un composé de formule (VI)

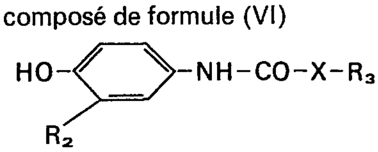

(VI)

dans laquelle $R_2$, $R_3$ et X ont la signification donnée ci-dessus, avec un composé de formule (VII)

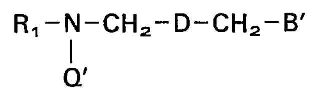

(VII)

dans laquelle

$R_1$ a la signification ci-dessus indiquée,

B' est un groupe réactif,

D représente un groupe CO ou $CH-OR''_4$, où $R''_4$ a la signification donnée ci-dessus pour $R_4$ ou bien représente, ensemble avec B', un trait de valence, et

Q' a la signification donnée ci-dessus pour Q ou bien représente, ensemble avec B', une liaison simple,

et, dans le cas où D est le groupe CO, on fait suivre une réduction, on élimine éventuellement le groupe de protection représenté par Q ou Q', on transforme éventuellement ultérieurement dans un composé obtenu de formule I un reste $R_4$ selon une méthode habituelle en un autre reste $R_4$, tel que défini dans la revendication 1, et on transforme éventuellement les composés obtenus en leurs sels pharmacologiquement tolérables.

8. Composés selon les revendications 1 à 6, pour combattre les maladies du cœur.

9. Médicaments, caractérisés en ce qu'ils ont une teneur en composés de formule générale I selon les revendications 1 à 6, ou leurs sels pharmacologiquement tolérables.